# EUROPEAN PATENT APPLICATION

(11) **EP 2 241 246 A1**
(43) Date of publication of application: **20.10.2010**
(21) Application number: 09157885.6
(22) Date of filing: 14.04.2009
(51) Int. Cl.: A61B 5/00, A61B 5/0488

(54) **Using muscle tension sensing to locate an analyte measurement site on the skin**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Apparatus for determining where to locate a measuring device (3) on skin, the apparatus having a muscle tension sensing device (1) arranged to sense muscle tension at different locations on the skin, and a processor (2) for determining and outputting an indication of where to locate the measuring device on the muscle, based on the sensed muscle tensions. This can help make the skin measurements such as non invasive glucose measurements more consistent, and more accurate.

## Description

### FIELD OF THE INVENTION

This invention relates to apparatus and methods for determining a location on the skin of an animal or human for an analyte concentration measuring site over a muscle by sensing or detecting the tensest muscle amongst a group of muscles, and to corresponding computer programs for carrying out such methods.

### BACKGROUND OF THE INVENTION

Many techniques have been investigated to detect skin analyte(s) concentration non-invasively by means of optical, electrical and/or optoelectronic methods, e.g., non-invasive glucose monitoring. Currently a number of companies are developing instruments for non-invasive glucose measurements based on optical methods. A major drawback or problem when measuring analytes based on optical methods comes from the varying optical properties of human skin tissue. Although these methods are proven to have sufficient sensitivity for in-vitro and/or ex-vivo glucose quantification, none of the currently existing companies have been successful in bringing a non-invasive device to the market. The main reason is that the accuracy and reliability of recently developed analyte measuring devices is not sufficient to get FDA approval.

Non-invasive analyte measurement is the most desirable method for consumers. But the uncertainty and inaccuracy hampers the acceptance of non-invasive testing. There is a need in the non-invasive glucose-monitoring market to solve the inaccuracy and unreliability problems.

Also methods and instruments have been developed for minimally invasive measurement of physiological parameters in a human or animal body, such as, for example, glucose measurements based on optical methods. These methods make use of a sensor, e.g., microsensors, implanted beneath the skin which is in contact with subcutaneous fluids. The sensor may include gels, particles, and/or liquids which are biodegradable. Preferably, the sensor, or microsensor, that is implanted is small in size, and does not require a complicated or painful insertion below the skin. The present invention has the capacity to increase the accuracy and reliability of the sensor or microsensor readout because it determines an optimum location on the skin to measure an analyte concentration by informing the user of the muscle direction/orientation relative to the analyte measuring device so the measurement can be taken.

Typically, analyte measurements methods deal with a large number of chemical, physical, and physiological interferences that obscure the true analyte concentration. These chemical, physical and physiological interferences include, *inter alia,* the water and salt concentrations in the skin, the level of melanin in the skin, the temperature of the skin, the movement of the skin, and whether the analyte measurement probe is correctly aligned with the muscle below the skin. This is true for both non-invasive and minimally invasive analyte measurement methods.

It is known that measurements performed on human tissue are often corrupted by large variations of the measurement sample that do not relate to the desired target analyte. These disturbing and undesired variations are called interferences to the target analyte. Often times these large variations are due to the chemical, physical and physiological interferences like the ones mentioned in the previous paragraph. One attempt to solve this issue is based on mathematical analyses of the measured data. Typical approaches apply chemometrical methods, (e.g., multivariate analysis), to extract the desired information from an analyte measurement that is corrupted by these various interferences. Basically these chemometrical methods are about performing post-processing of the measure data to filter out the interferences corrupting the target analyte concentration. However, for such complex systems as human skin, a large number of interferences can have a large influence on the measured analyte concentration. Therefore, post-processing chemometric methods become very complex and are prone to large errors. The instant invention discloses a way to stabilize the human skin, (or the measurement volume), during the analyte measurement itself. The apparatus and methods of the current invention can reduce or eliminate some of these interferences by informing the user, based on the strongest EMG signals, when the analyte measuring device is directly aligned with the muscle and the measurement can be taken. Thus, the error correction for these interferences is moved from the post-processing phase into the signal acquisition phase. This will increase the reproducibility and accuracy of non-invasive and minimally invasive analyte measurements taken from the skin.

### SUMMARY OF THE INVENTION

An object of the invention is to provide apparatus and methods for determining an optimum location for an analyte measuring site over a muscle by sensing muscle tension, and corresponding computer programs for carrying out such methods. According to a first aspect, the invention provides:
Apparatus for determining where to locate an analyte measuring device in, on or under the skin, the apparatus having a muscle tension sensing device arranged to sense muscle tension at different locations on the skin, and a processor for determining and outputting an indication of where to locate the measuring device on the muscle, based on the sensed muscle tensions.

This can help make the skin measurements of analytes more consistent, and more accurate. The analyte measuring device can include a microsensor for location subcutaneously under the skin for minimally-invasive analyte testing.

Embodiments of the invention can have many other features added; some such additional features are set out in dependent claims and described in more detail below.

Other aspects of the invention include corresponding methods, and computer programs for processing the muscle tension sensing signals to determine and output the indication of where to locate the analyte measuring device over the muscle. In particular the present invention relates to a computer program product that, when executed on a processing engine, implements any of the methods or the apparatus of any of the embodiments of the present invention. The computer program product can be stored on any suitable machine readable signal storage device such as magnetic disk memories such as diskettes or hard drives, optical storage media such as CDROM or DVD ROMs, magnetic tape media or solid state memories such as USB sticks.

Many of the additional features can be combined together and combined with other aspects of the invention. Other advantages will be apparent to those skilled in the art, especially over the prior art. Numerous variations and modifications can be made without departing from the claims of the present invention. Therefore, it should be clearly understood that the form of the present invention is illustrative only and is not intended to limit the scope of the present invention.

### BRIEF DESCRIPTION OF THE DRAWINGS:

How the present invention may be put into effect will now be described by way of example with reference to the appended drawings, in which:
FIG. 1 shows a schematic view of an apparatus according to an embodiment,
FIG. 2 shows a schematic view of a muscle tension sensing array.
FIG. 3 shows EMG signals from electrode pair 1 (black) and pair 2 (grey) when the muscle is relaxing.
FIG. 4 shows EMG signals from electrode pair 1 (black) and pair 2 (grey) when the muscle is slightly contracted/tensed.
FIG. 5 shows another embodiment.
FIG. 6 is another embodiment involving stimulating the muscle.
FIG. 7 shows a method according to an embodiment.

### DETAILED DESCRIPTION OF EMBODIMENTS:

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. Where the term "comprising" is used in the present description and claims, it does not exclude other elements or steps. Where an indefinite or definite article is used when referring to a singular noun e.g., "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention. References to a signal can encompass any kind of signal in any medium, and so can encompass an electrical or optical or wireless signal or other signal for example. References to analyzing can encompass processing a signal in any way to derive or enhance information about the material. References to a processor can encompass any means for processing signals or data in any form and so can encompass for example a personal computer, a microprocessor, analog circuitry, application specific integrated circuits, software for the same, and so on.

In the description provided herein, numerous specific details are set forth. However, it is understood that embodiments of the invention may be practiced without these specific details. In other instances, well-known methods, structures and techniques have not been shown in detail in order not to obscure an understanding of this description.
Additionally, the term, and all forms of the term, "sense" or "sensing" is used synonymously herein with the term, and all forms of the term "detect" or "detecting."

### Introduction to some issues addressed by some of the embodiments

By way of introduction to the embodiments, the problem of measuring analytes through the skin, in particular, minimally invasive and non invasive blood glucose monitoring will be discussed briefly. The measurement of glucose through near-infrared spectroscopy is based on a change in the concentration of glucose being indicated by a change in the absorption of light according to the absorption and scattering properties of glucose and/or the effect of glucose changes upon the anatomy and physiology of the sampled site. However, in addition to the effect of glucose on the near-infrared light probing signal that is delivered to the skin to determine its concentration, the probing signal is also reflected, diffusely reflected, transmitted, scattered, and absorbed in a complex manner related to the structure and composition of the tissue. When near-infrared light is delivered to the skin, a proportion of reflected light, or specular reflectance, is typically between 4-7% of the delivered light over the entire spectrum. Absorption by the various skin constituents accounts for the spectral extinction of the light within each layer. Scattering is the main process by which the beam may be returned to contribute to the diffuse reflectance of the skin. Scattering also has a strong influence on the light that is diffusely transmitted through a portion of the skin.

The scattering of light in tissues is partly due to discontinuities in the refractive indices on the microscopic level, such as the aqueous-lipid membrane interfaces between each tissue compartment or the collagen fibrils within the extracellular matrix. The spectral characteristics of diffuse remittance from tissue result from a complex interplay of the intrinsic absorption and scattering properties of the tissue, the distribution of the heterogeneous scattering components, and the geometry of the point(s) of irradiation relative to the point(s) of light detection.

The near-infrared absorption of light in tissue is primarily due to overtone and combination absorbances of the C-H, N-H, and O-H functional groups. As skin is primarily composed of water, protein, and fat; these functional groups dominate the near-IR absorption in tissue. As the main constituent, water dominates the near-infrared absorbance above 1100 nm and is observed through pronounced absorbance bands at 1450, 1900, and 2600 nm. Protein, in its various forms, in particular, collagen is a strong absorber of light that irradiates the dermis. Near-infrared light that penetrates to subcutaneous tissue is absorbed primarily by fat. In the absence of scattering, the absorbance of near-infrared light due to a particular analyte, A, can be approximated by Beer's Law. An approximation of the overall absorbance at a particular wavelength is the sum of the individual absorbance of each particular analyte given by Beer's Law. The concentration of a particular analyte, such as glucose, can be determined through a multivariate analysis of the absorbance over a multiplicity of wavelengths because it is unique for each analyte. However, in tissue compartments expected to contain glucose, the concentration of glucose is at least three orders of magnitude less than that of water. Given the known extinction coefficients of water and glucose, the signal targeted for detection by reported approaches to near-infrared measurement of glucose, i.e., the absorbance due to glucose in the tissue, is expected to be, at most, three orders of magnitude less than other interfering tissue constituents. Therefore, the near-infrared measurement of glucose requires a high level of sensitivity over a broad wavelength range. Multivariate analysis is often utilized to enhance sensitivity, but often this sensitivity is still inadequate for accurate and reliable analyte measurement.

In addition, the diverse scattering characteristics of the skin, e.g., multiple layers and heterogeneity, cause the light returning from an irradiated sample to vary in a highly nonlinear manner with respect to tissue analytes, in particular, glucose. Simple linear models, such as Beer's Law have been reported to be invalid for the dermis.

Dynamic properties of the skin also add to the difficulties. Variations in the physiological state and fluid distribution of tissue profoundly affect the optical properties of the tissue layers and compartments over a relatively short period of time. Therefore, for all these reasons, the optical properties of the tissue sample are modified in a highly nonlinear and profound manner that introduces significant interference into noninvasive and minimally invasive analyte measuring on or in the skin.

The measurement of glucose through spectroscopy can also be made by a change in the absorption of light according to the absorption and scattering properties of minimally invasive microsensors, or to the change in light emitted or reflected from such microsensors located below the skin. Such methods using microsensors may include, for example,
- observing fluorescence (e.g., fluorescence resonance energy transfer) of a competitive binding assay encapsulated in microcapsules, for example based on competitive binding between the protein Concanavalin A and various saccharide molecules, specifically a glycodendrimer and glucose, the microcapsules can be polyelectrolyte microcapsules;
- detecting glucose using boronic acid-substituted viologens in fluorescent hydrogels in which a fluorescent anionic dye and a viologen are appended to boronic acid, which serve as glucose receptors, and are immobilized into a hydrogel, the fluorescence of the dye being modulated by the quenching efficiency of the viologen based receptor which is dependent upon the glucose concentration; - other methods, e.g., monitoring oxygen or pH or other "smart tattoo" related methods.

Another interference related to measuring an analyte in the blood, either non invasively or minimally invasively through the skin is the fact that the skin is a supple tissue. It tends to move while placing a measuring device on or in it. This skin movement makes accurate measurement of analytes such as glucose more difficult. It has now been discovered that skin movement depends on skin position relative to muscle position and direction. It is rather difficult for users, whether they are medical practitioners, or test subjects, to identify the optimum location to place the analyte measuring device over an embedded sensor, for example, to measure the analyte concentration while the muscle is relaxed, or the group of muscles are relaxed, for the most accurate and reliable concentration measurement. This is due in part because a user cannot "see" or determine a muscle's direction or orientation when the muscle is relaxed. If a user cannot determine the true orientation or direction of the muscle where the sensor is located then it is difficult to accurately and reliably align the analyte measuring device to read the analyte concentration. The direction and orientation is much easier to determine for a tensed muscle than for a relaxed muscle with the aid of a muscle tension sensing device indicating which muscle is tensed relative to the surrounding muscles. This tensed muscle also minimizes skin movement so that a more accurate and consistent analyte measurement can be taken.

### Introduction to features of the embodiments

To reduce the skin movement that influences the accuracy and reliability of determining analyte concentrations in the blood, the analyte measuring device can be aligned with the muscle as described below. Embodiments of this invention enable more accurate and consistent placement of the measuring device by using a muscle tension sensing array over the required measuring site on the body. Embodiments can have any kind of muscle tension sensing device arranged to sense muscle tension at different locations on the skin. Another feature is a processor of any kind for determining and outputting an indication of where to place the analyte measuring device over the muscle that shows the strongest EMG signal identified by the muscle tension sensing device.

The processor can comprise an electromyographic device. The muscle tension sensing device can have an array of two or more pairs of electrodes at different places on the skin, with each member of the pair placed parallel to each other, but always placed perpendicular relative to the underlying muscles, with the second pair of electrodes placed adjacent to the first and so on for subsequent electrode pairs, the processor being arranged to determine and indicate which pair of electrodes shows the greatest muscle tension relative to the surrounding muscles. The apparatus can be arranged to electrically stimulate a particular muscle to induce muscle tension to stabilize the overlying skin. Then the processor can be arranged to determine the muscle direction/orientation of the muscle eliciting the strongest EMG signal relative to the surrounding muscles, and project the optimum placement on the skin, to enable the analyte measuring location to be aligned with the muscle direction.

The apparatus can have an indicator part for indicating on the skin itself the optimum location to place the analyte measuring device on the skin. The indicating part which projects the optimum location on the skin can be integrated with the muscle tension sensing device. The apparatus can include a part of the analyte measuring device in the form of a hand held minimally invasive or non invasive optical probe, suitable for a user to align the probe on the skin according to the indication. The probe can be integrated with the muscle tension sensing device, or with a part for indicating the proper location. The location indicator can project onto the skin or indicate by an intuitive interface the proper position and/or orientation for the analyte measuring device to be maximally aligned with the muscle showing the strongest EMG signal.

A method of measuring characteristics of the skin such as wrinkles, water content, protein composition or level of protein breakdown, (e.g., collagen and/or other proteins involved in maintaining skin integrity), involves sensing muscle tension at different locations on the skin as for analyte measuring, determining and outputting an indication of where on the skin to locate the measuring device, based on the muscle with the highest muscle tension relative to its adjacent muscles, and taking the measurement of the characteristic based on the location indicated. The measuring can involve moving a hand held non invasive optical probe on the skin to align it according to the indication. The measuring can involve measuring reflectance and retrieving an output about the particular characteristic in the skin, based on that reflectance.

Embodiments of the present invention also relate to a method of measuring a characteristic of skin by sensing muscle tension at different locations on the skin, determining and outputting an indication of where on the skin to locate a microsensor for minimally invasive diagnostics, based on the muscle with the highest muscle tension relative to its adjacent muscles, and taking the measurement of the characteristic based on the location indicated.

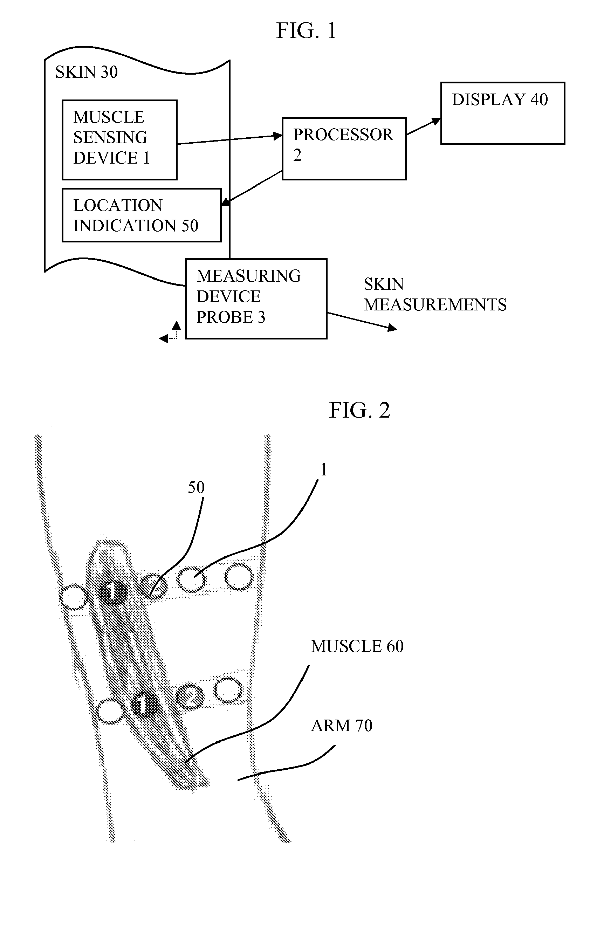

### FIG. 1, first embodiment of the invention

FIG. 1 shows a schematic view of an apparatus according to an embodiment. On a surface of the skin 30 is a muscle sensing device 1, which may be in the form of electrodes on a pad stuck to the skin. Other types of device can also be envisaged. Muscle tension sensing signals are sent to a processor 2 for determining the muscle position and direction. Examples of sensing signals are described below with reference to FIG.'s 3 and 4. The processor determines and outputs an indication of a proper location on the skin for the measuring device probe 3 which is used for measuring a blood analyte such as glucose or a characteristic of the skin such as skin health and/or protein content such as collagen. This indication can be on a display 40 or in some form on the skin itself, as shown by location indication 50. This indication could be, for example, a projected image on the skin, an array of LEDs on a skin patch, or an indication on the probe, to show a user which direction to move the measuring device in order to approach the best position to measure the analyte or characteristic.

While the apparatus of the invention is in use, the muscle tension can be induced either by user actions, or by fast electrical stimulation provided by the muscle tension sensing array. The resulting muscle tension signals are recorded by the muscle tension sensing array. The electrode pair of the sensing array which generated the strongest signal is distinguished from the adjacent muscles which also have electrode pairs placed thereupon, and the location of this muscle is registered via an appropriate algorithm. The registered position should be the optimum position which aligns over the muscle with the strongest EMG signal, thereby the most tense muscle relative to the adjacent muscles thereby revealing the best measuring site. A reduced skin movement at the proper position is expected because the muscle is tense. Experimental proof provides evidence for the high correlation between muscle tension signals and positions of the electrode pair from the muscle tension sensing array. It indicates that the proper position which aligns with the muscle can be recognized by a muscle tension sensing system, e.g., EMG sensor array.

Such apparatus can simplify the identification of the proper alignment of the analyte measuring device, (or skin characteristic measuring device), over an embedded analyte concentration or diagnostic biosensor by indicating the most tense muscle in the area, and hence the most stable skin location. As a result, the analyte or skin characteristic measurement can more accurate and reliable because the measuring device is placed directly over the biosensor and the skin is stable. As described, this and other embodiments are notable in that:
- Muscles tension/contraction is monitored by sensing array, e.g., EMG sensor array.
- The muscle tension is induced either from human actions or stimulated by electrical pulses.
- The muscle direction is identified by the muscle tension sensing array, via an appropriate algorithm.
- The muscle tension sensing device is placed aligned with the muscle.
- The analyte or skin characteristic measuring device can be a minimally invasive device.

The consequences can include:
- Ease of identifying proper position by muscle tension sensing array,
- Enabling placement of the analyte or skin characteristic measuring device aligned with the most tense muscle,
- Reducing the tissue movement during measurement.

### EMG systems

It is known to use EMG to use electrodes to stimulate muscles, focusing on pain relief. Reference is made to these patents for details of conventional methods to implement such EMG sensing. For example US Pat. 4570640 "Sensory monitoring apparatus and method" shows monitoring the sensory system of a patient to enable determination of the level and depth of spinal and epidural nerve blocks, including those induced by anesthetics administered to a patient, which blocks affect the sympathetic and motor nervous systems. The anesthesia level is sequentially and repeatedly scanned at a plurality of spaced points to provide a continuous determination of the extent and depth of superficial and deep sensation and sympathetic and motor integrity. The electronic apparatus includes a stimulator that provides selective stimulation to each element of a multiple element transmitting unit the elements of which are non-invasively positioned contiguous to the skin of a patient. A physiological response detector is used to detect patent responses to stimulation sensed by the elements of a multiple element sensing unit the elements of which are also non-invasively positioned contiguous to the skin of the patient. A multiple display is provided to facilitate monitoring and an indication and/or termination of stimulation signal is provided whenever stimulation exceeds a reference level.

Reference is also made to US Pat. 6600954, entitled, "Method and apparatus for selective control of nerve fibers", which shows using a plurality of electrode devices spaced along the length of the nerve bundle which are sequentially actuated with delays corresponding to the velocity of propagation of the body-generated action potentials through the large-diameter fibers to produce a "green wave" effect which minimizes undesired anodal blocking of the large-diameter fibers while maximizing the collision blocking of the small-diameter fibers.

Reference is also made to EP 1637076 which shows detecting and processing EMG signals using a substrate having a bottom surface adapted for attachment to skin; a plurality of spaced apart electrode arrays projecting from the bottom surface so as to engage the skin and detect EMG signals in muscles located under the substrate; and four differential amplifiers connected to receive EMG signals from four distinct pairs of electrode arrays. The electrode arrays detect the action potentials of the muscle fibers from various orientations so that the shape of an action potential appears substantially dissimilar in each of the four differential pairs.

The EMG signal is composed of the action potentials (or electrical pulses) from groups of muscle fibers (grouped into functional units called motor units). Reference can be made to the book "Muscles Alive" (5 Th.Ed, 1985) for more details. The EMG signal is detected with electrodes placed on the surface of the skin or with needle or wire electrodes introduced into the muscle tissue. The term decomposition is commonly used to describe the process whereby individual motor unit action potentials (MUAPs) are identified and uniquely classified from a set of superimposed motor unit action potentials which constitute the EMG signal. A decomposed EMG signal provides all the information available in the EMG signal. The timing information provides a complete description of the inter-pulse interval, firing rate and synchronization characteristics. The morphology of the shapes of the MUAPs provides information concerning the anatomy and health of the muscle fibers.

The above embodiments may also be used for minimally invasive apparatus and methods.

### FIG.'s 2, 3, 4, another embodiment

FIG. 2 shows an arrangement of electrodes to form an array on a patch attached to an arm 70. EMG signals from this sensing array, consisting of pairs of "stick-on" electrodes, placed over the forearm such that each member of the pair is parallel to each other, and adjacent to the next pair, are sent to a processor in the form of a computer and Moby8, a multi-channel biological signal processing device (TMSi, Twente). The sampling frequency is 2048 Hz, using high pass cut off filter of 6 Hz.

EMG signals are produced from user-induced muscle contraction/tension. Various EMG signals are acquired from pairs of electrodes, e.g., pair 1 and pair 2 as shown in FIG. 2, which are placed with the same orientation with respect to the muscle shown by item 60. The reference electrodes are not shown in FIG. 2 for the sake of clarity.

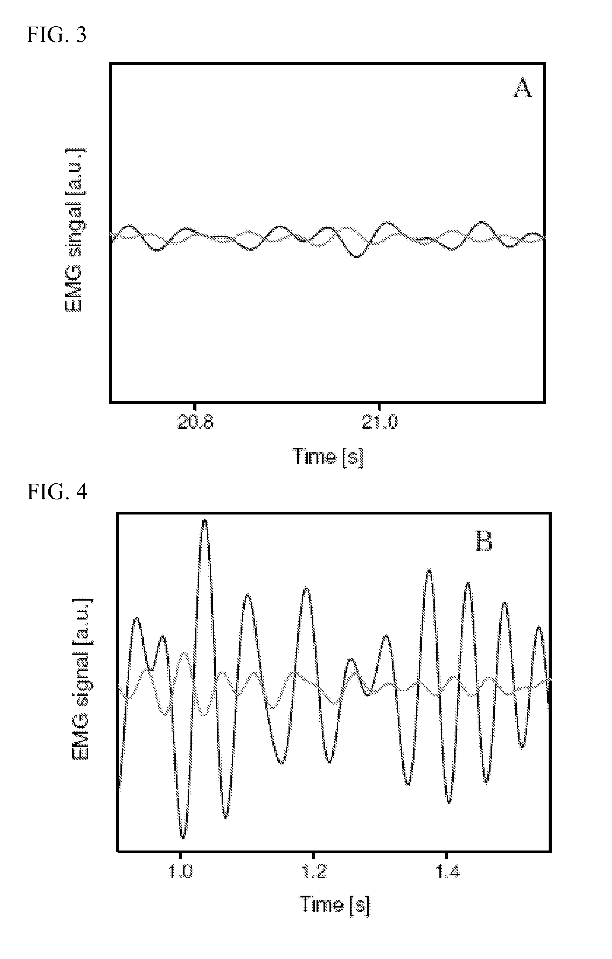

The sensing array was placed around the measuring site, where pair 1 of the electrodes was placed on the muscle and pair 2 of the electrodes was placed adjacent to the muscle of interest, as shown in FIG. 2. The EMG signals from electrode pair 1 and pair 2 are both shown in the graphs depicted in FIG.'s 3 and 4. No obvious difference was detected between electrode pair 1 and pair 2 when the subject was asked to relax (FIG. 3). However significantly different signals from electrode pair 1 and pair 2 were observed when the subject was asked to contract his muscles gently (FIG. 4). A much stronger EMG signal from electrode pair 1 is observed when compared with the signal from pair 2. FIG.'s 3 and 4 provide an example of how large the difference can be between muscles in the EMG signals when the extremity is relaxed and the extremity is tensed. The amplitude of the EMG signal can be quantified in Volts, e.g., µV, mV; in percentage of MVIC, in Mean amplitude of the rectified signal and/or RMS value of the signal.

The results shown in the graphs illustrate a high correlation between EMG signal amplitude and the position of the electrode pairs when the muscle is tensed. It proves the principle that the position and orientation of an individual muscle can be identified out of a group of muscles by the muscle tension sensing array.

The above embodiments may also be used for minimally invasive apparatus and methods.

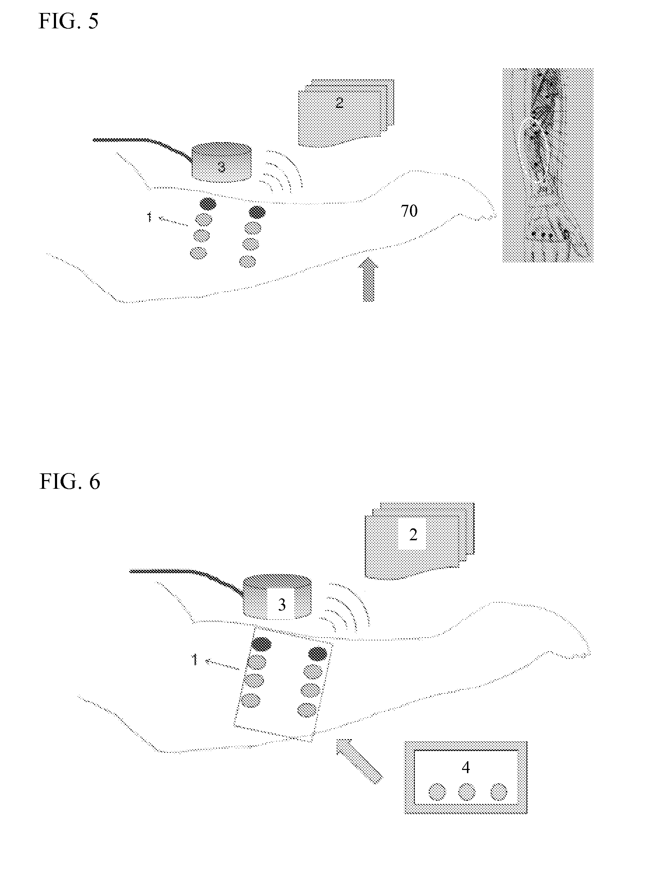

### FIG. 5, another embodiment

FIG. 5 shows an example of an apparatus according to an embodiment. It shows a means to identify a specific muscle as indicated in the inset. The inset shows a view of parts of an arm 70, with a particular muscle highlighted by being encircled. The electrode array (1) has four pairs of electrodes on one or more patches applied to the arm. There may be many more electrodes and they may be in different orientations. The electrodes feed signals to a processing unit (2) which measures the EMG signals when the wrist is lifted.
These signals are interpreted by algorithms of the processing unit (2). The pair of electrodes which generates the strongest EMG signal amongst the sensing array is distinguished by the processing unit (2), via an appropriate algorithm. The indication of which electrodes provide the strongest signal can be displayed on a separate display, or on some display means such as small LEDs integrated alongside each electrode in the patch on the skin. An alternative way of indicating the optimum location to measure the analyte or skin characteristic would be to provide an indication on the probe 3 as to which direction it should be moved to be in the correct position to measure the analyte or skin characteristic.

The analyte or skin characteristic measurement device (3) such as an optical probe of a non invasive optical reflectance system is then placed at the proper site over the muscle with the strongest EMG signal as indicated. The measurement can then be made at the appropriate location on the body with less interference from skin movement because the underlying muscle is tenser than its neighbors.

The above embodiments may also be used for minimally invasive apparatus and methods.

### FIG. 6, another embodiment

Figure 6 shows an embodiment similar to that of FIG. 5 and corresponding reference numerals are used as appropriate. In this case there is also a means for electrically stimulating the muscle to reduce muscle movement for a more accurate analyte measurement. An electrode array (1) is placed around the measuring site, e.g., the forearm as described in FIG. 5, which functions as the stimulating element as well as the sensing element by using differentiated frequency filters for example. A fast electrical stimulation (for example, about 100 ms to avoid uncomfortable sensation) is provided by a generator (4) which is coupled to the electrode array (1) to induce tension of the muscle. Meanwhile the muscle tension signals are recorded by the sensing array (1), and are interpreted and sent to the processor (2). The pair of electrodes which detect the strongest EMG signal, indicating the tensest muscle among a group of muscles, being monitored by the muscle tension sensing array is distinguished by the processor (2), via an appropriate algorithm. The measuring device (3) is placed at the proper site and the analyte or skin characteristic measurement can then be made at the appropriate location with less interference from skin movement.

In some cases, the movement of the analyte or skin characteristic measuring device can be driven automatically by the processor to reach the optimum determined position, rather than using a hand held device. In some cases, the tension sensing device can be incorporated in a handheld device together with the probe, and with an indicator to indicate when the probe is in the best position to measure the analyte concentration or the skin characteristic. Another embodiment can be arranged to take a number of measurements at different identifiable locations and use the indication to select which of the measurements to use, rather than using it to move the probe. In another embodiment, a number of sensing signals from different locations can be interpolated to give a more accurate location indication, optionally with both position and orientation information.

The above embodiments may also be used for minimally invasive apparatus and methods.

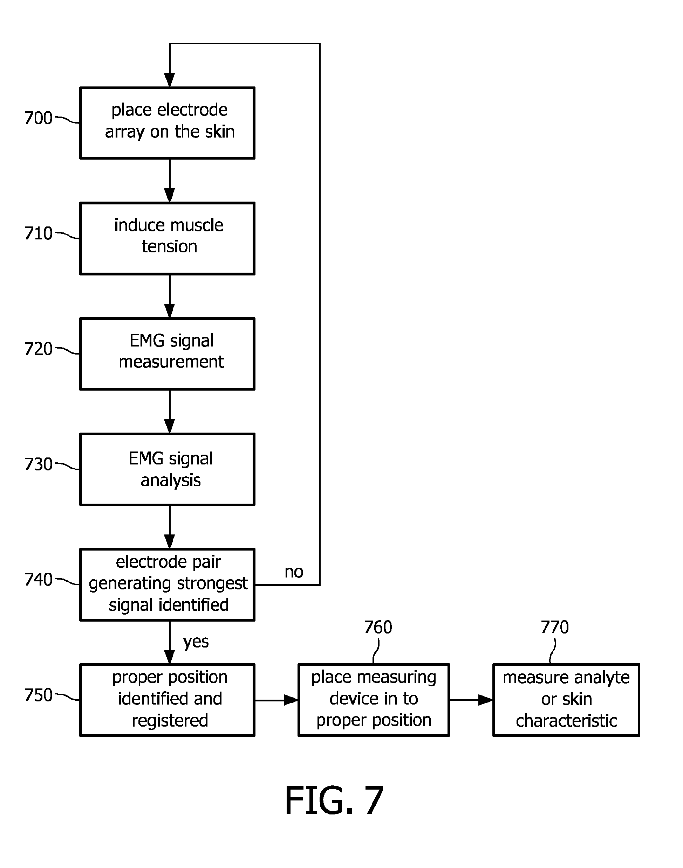

### FIG. 7, method embodiment

FIG. 7 shows steps in a method using the apparatus of FIG.'s 5 and 6 or other embodiments. At first step 700, the array of electrodes is placed on the body site perpendicular to the muscle group of the body. Next, at step 710, muscle tension is induced either by user actions, or by fast electrical stimulation and at step 720 the resultant muscle tension signals, as EMG signals, are measured by the muscle tension sensing array. At step 730 the EMG signals are analyzed and at step 740 if the electrode pair of the sensing array which generated the strongest signal can be distinguished from the EMG signal generated by the surrounding muscles then the analyte, or skin characteristic, measuring device position is properly identified and is registered via an appropriate algorithm at step 750. If the electrode pair of the sensing array which generated the strongest signal cannot be distinguished from the EMG signals generated from the surrounding muscles at step 740, then the algorithm returns to step 700 to perform steps 700-740 until the electrode pair of the sensing array which generated the strongest signal can be distinguished from the signals generated by the surrounding muscles, and the analyte, or skin characteristic, measuring device position is properly identified and is registered via an appropriate algorithm at step 750. At step 760 the user places the analyte or skin characteristic measuring device at the proper location and measures the analyte concentration or skin characteristic at step 770.

The above embodiments may also be used for minimally invasive apparatus and methods.

### Applications

The Apparatus as proposed here, can be used by most techniques that provide analyte concentrations or other skin measurements within any kind of skin. Embodiments of the invention can be used for non-invasive glucose concentrations by means of NIR diffuse back-reflectance spectroscopy. Further applications include measurements of skin properties, (e.g., inter alia, skin cancer, skin aging, skin health, protein content, water content, etc.), by means of light, or any measurements susceptible to interference by skin movement. Any of these applications may also be used with minimally invasive methods.
Other variations can be envisaged within the scope of the claims.

## Claims

1. An apparatus for determining where to locate an analyte measuring device (3) in, on or under the skin, the apparatus having a muscle tension sensing device (1) arranged to sense muscle tension at different locations on the skin, and a processor (2) for determining and outputting an indication of where to locate the measuring device on the muscle, based on the sensed muscle tensions.

2. The apparatus of claim 1, wherein the processor comprises an electromyographic device.

3. The apparatus of claim 1 or 2, wherein the sensing device comprises an array (1) of two or more pairs of electrodes at different places on the skin, the processor being arranged to determine and indicate which pair of electrodes shows greater muscle tension.

4. The apparatus of any preceding claim wherein the apparatus is arranged to stimulate the muscle to detect the tension.

5. The apparatus of any preceding claim, wherein the processor is arranged to determine a muscle direction on the skin, to enable the analyte measuring location to be aligned with the muscle direction.

6. The apparatus of any preceding claim, comprising an indicator part (50) for indicating on the skin the location for the analyte measuring device on the skin.

7. The apparatus of claim 6, wherein the indicating part is integrated with the muscle tension sensing device.

8. The apparatus of any preceding claim comprising a part of the analyte measuring device in the form of a hand held non invasive optical probe, suitable for a user to align on the skin according to the indication.

9. A method of measuring a characteristic of skin, the method having the steps of sensing muscle tension at different locations on the skin, determining and outputting an indication of where on the skin to locate the measurement of the characteristic, based on the sensed muscle tensions, and making the measurement of the characteristic based on the location indicated.

10. The method of claim 9 wherein the sensing step uses electromyographic sensing.

11. The method of claim 9 or 10, wherein the sensing step involves placing an array of two or more pairs of electrodes at different places on the skin, and the indication detecting which of the electrode pairs show greater muscle tension.

12. The method of claim 9, 10 or 11, wherein the sensing step involves stimulating the muscles to increase the muscle tension of one muscle compared to the surrounding muscles.

13. The method of any of claims 9 to 12, wherein the measuring involves moving a hand held non invasive optical probe on the skin to align it according to the indication.

14. The method of any of claims 9 to 13, where in the measuring involves measuring the reflectance and determining a level of glucose in the skin, based on the reflectance.

15. A computer program product that when executed on a processing engine, implements any of the methods of claims 9 to 14 or the apparatus of any of the claims 1 to 8.
